# EUROPEAN PATENT APPLICATION

(11) **EP 4 600 171 A1**
(43) Date of publication of application: **13.08.2025**
(21) Application number: 24833793.3
(22) Date of filing: 29.11.2024
(51) Int. Cl.: B65D 33/16, B65D 43/02, B65D 77/04, B65D 45/16

(54) **TRANSPORT BAG AND TRANSPORT BOX**

(30) Priority: 29.11.2023 US 202363603737 P
(71) Applicant: Wong, Cheung Hing, Kowloon, Hong Kong (HK)
(72) Inventor: Wong, Cheung Hing, Kowloon, Hong Kong (HK)
(74) Representative: Isern Patentes y Marcas S.L.
(86) International application number: PCT/CN2024/135448
(87) International publication number: WO 2025/113600

(57) **Abstract**

The embodiment of the present application relates to the technical field of transportation tools for medical equipment, and particularly discloses a transport bag and a transport box, which comprise a bag body provided with an accommodating cavity and a bag opening communicated with the accommodating cavity. The bag body comprises first and second parts connected with each other, a first folding side is provided between the first and second parts, the first and second parts are folded and connected with each other along the first folding side, the bag opening is located on the first part, the first part comprises first connecting sides, the second part comprises second connecting sides, and one of the second connecting sides is correspondingly connected with one of the first connecting sides so that the bag body forms a plurality of connecting edges. In the aforesaid way, the embodiment of the present application can avoid the gap between the transport bag and the tray opening, thereby reducing the risk of contamination of endoscopes or other medical equipment to be transported.

## Description

### Technical field

The present application relates to the technical field of transportation tools for medical equipment, and in particular, relates to a transport bag and a transport box.

### Background

An endoscope is a kind of tube equipped with a light source, which can peep into the internal conditions of a human body or an object through its natural pores, and thus it is widely used in medical and industrial fields. As a medical instrument, the medical endoscope plays an important role in clinical observation and diagnosis by inserting into various cavities in the human body to peep at lesions of internal organs. In the prior art, during the transportation of an endoscope, the endoscope is usually placed on a tray and covered with a transport bag with two closed edges, and the two closed edges cover the tray.

In the process of realizing the present application, the inventor of the present application found that the transport bag in the related art only has two closed sides covering the tray, and during the transportation, a gap is likely to appear between the transport bag and the tray opening, so that the inside of the tray is likely to communicate with the outside through the gap, thereby increasing the risk of contamination for the endoscope.

### Summary

In view of the above problems, an embodiment of the present application provides a transport bag and a transport box so that the above problems are overcame or partially solved.

According to one aspect of the present application, a transport bag is provided, which comprises: a bag body, being provided with an accommodating cavity and a bag opening communicated with the accommodating cavity; wherein, the bag body comprises a first part and a second part which are connected with each other, a first folding side is provided between the first part and the second part, the first part and the second part are folded and connected with each other along the first folding side, the first part comprises a plurality of first connecting sides, the second part comprises a plurality of second connecting sides, and one of the second connecting sides is correspondingly connected with one of the first connecting sides so that the bag body forms a plurality of connecting edges.

In an optional mode, the plurality of first connecting sides include a first upper side, a first lateral side and a first lower side, the first lateral side is located between the first upper side and the first lower side; the plurality of second connecting sides include a second upper side, a second lateral side and a second lower side, the second lateral side is located between the second upper side and the second lower side, and the second upper side is connected with the first upper side, the second lateral side is connected with the first lateral side, and the second lower side is connected with the first lower side.

In an optional mode, the transport bag at least meets one of the following conditions: the first upper side and the second upper side are in hot-press sealing connection; the first lateral side and the second lateral side are in hot-press sealing connection; and the first lower side and the second lower side are in hot-press sealing connection.

In an optional mode, the first part and the second part are symmetrically arranged with respect to the first folding side.

In an optional mode, the outer surface of the second part is further provided with a first identification symbol.

In an optional mode, the bag body is made of flexible materials.

According to another aspect of the present application, a transport box is provided which comprises a box body and a cover plate, wherein the box body is provided with an accommodating space and an opening communicated with the accommodating space, the cover plate covers the opening, and the periphery of the cover plate is connected with the periphery of the box body.

In an optional mode, the cover plate comprises a first surface and a second surface which are oppositely arranged, and when the cover plate covers the opening, the first surface or the second surface is located in the accommodating space.

In an optional mode, the first surface is provided with a second identification symbol, and the second surface is provided with a third identification symbol.

In an optional mode, the periphery of the box body extends with a stepped part in the direction away from the accommodating space, and one side of the stepped part away from the cover plate is provided with a clamping protrusion, and a clamping groove is arranged between the clamping protrusion and the outer wall of the box body; the periphery of the cover plate is further provided with a fastening plate, the fastening plate is rotatably connected to the cover plate and can rotate relative to the cover plate, one side of the fastening plate close to the first surface is provided with a first hook, and the other side of the fastening plate close to the second surface is provided with a second hook, wherein when the first surface is located in the accommodating space, the first hook is clamped in the clamping groove, and the first hook abuts against the clamping protrusion; and when the second surface is located in the accommodating space, the second hook is clamped in the clamping groove, and the second hook abuts against the clamping protrusion.

Beneficial effects of the embodiment of the present application lie in that: unlike the situation in the prior art, the embodiment of the present application is provided with a bag body which is provided with an accommodating cavity and a bag opening communicated with the accommodating cavity, the bag body comprises a first part and a second part which are connected with each other, a first folding side is provided between the first part and the second part, the first part and the second part are folded and connected with each other along the first folding side, the bag opening is located on the first part, the first part comprises a plurality of first connecting sides, the second part comprises a plurality of second connecting sides, and one of the second connecting sides is correspondingly connected with one of the first connecting sides so that the bag body forms a plurality of connecting edges. When users need to transport endoscopes or other medical equipment, they only need to put the endoscopes or other medical equipment to be transported on the tray, then cover the tray from the bag opening until the tray is located in the accommodating cavity, and make the plurality of connecting edges on the bag body attach to the periphery of the tray so that the tray is located in the enclosed accommodating cavity and thus the endoscopes or other medical equipment located in the tray are located in the enclosed accommodating cavity . With this arrangement, the plurality of connecting edges of the transport bag all cover the tray so that a gap between the transport bag and the tray opening can be avoided, thereby reducing the risk of contamination of the endoscopes or other medical equipment to be transported.

### Brief description of the drawings

In order to explain specific embodiments of the present application or technical solutions in the prior art more clearly, attached drawings needed in the description of the specific embodiments or the prior art will be briefly introduced below. In all the attached drawings, like elements or parts are generally identified by like reference numerals. In the attached drawings, elements or parts are not necessarily drawn to actual scale.
Figure 1 is a schematic view at a certain angle of the overall structure of a transport bag according to an embodiment of the present application.
Figure 2 is a schematic view of a use state for the overall structure of a transport bag according to an embodiment of the present application.
Figure 3 is a schematic view of another use state for the overall structure of a transport bag according to an embodiment of the present application.
Figure 4 is a schematic exploded view at a certain angle of the partial structure of a transport box according to an embodiment of the present application.
Figure 5 is a schematic exploded view at another angle of the partial structure of a transport box according to an embodiment of the present application.

### Detailed description

In order to facilitate the understanding of the present application, the present application will be described in more detail below with reference to the attached drawings and specific embodiments. It shall be noted that when an element is expressed to be "fixed" to another element, it may be directly on the other element, or there may be one or more intervening elements therebetween. When an element is expressed to be "connected" to another element, it may be directly connected to the other element, or there may be one or more intervening elements therebetween. The terms "vertical", "horizontal", "left" and "right" and similar expressions used in this specification are for illustrative purposes only.

Unless otherwise defined, all technical and scientific terms used in this specification have the same meaning as commonly understood by those skilled in the art of this application. The terms used in the specification of the present application are only for the purpose of describing specific embodiments, and are not intended to limit the present application. The term "and/or" as used in this specification includes any and all combinations of one or more associated items listed.

In addition, the technical features involved in different embodiments of the present application described below can be combined with each other as long as they do not conflict with each other.

It shall be noted that, objects to which the technical solution of the present application is applied include, but are not limited to, endoscopes, scalpels, scissors, tweezers, catheters, injection heads, gauze, or the like as well as other medical equipment, and the application to endoscopes is described for illustration in the present application.

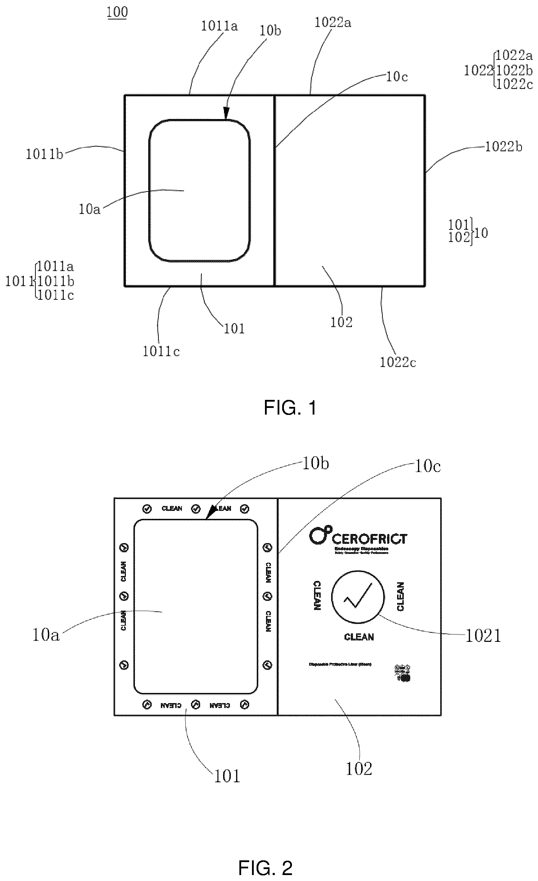

Referring to Figure 1, the transport bag 100 includes a bag body 10 that is provided with an accommodating cavity 10a and a bag opening 10b communicated with the accommodating cavity 10a, an endoscope to be transported is placed on a tray, and the transport bag 100 can cover the tray from the bag opening 10b.

As for the above-mentioned bag body 10, as shown in Figure 1 and Figure 2, the bag body 10 includes a first part 101 and a second part 102 which are connected with each other, a first folding side 10c is provided between the first part 101 and the second part 102, and the first part 101 and the second part 102 are folded and connected with each other along the first folding side 10c. The bag opening 10b is located on the first part 101, the first part 101 includes a plurality of first connecting sides 1011, the second part 102 includes a plurality of second connecting sides 1022, and one of the second connecting sides 1022 is correspondingly connected with one of the first connecting sides 1011 so that the bag body 10 forms a plurality of connecting edges. When users need to transport endoscopes or other medical equipment, they only need to put the endoscopes or other medical equipment to be transported on the tray, then cover the tray from the bag opening 10b until the tray is located in the accommodating cavity 10a, and make the plurality of connecting edges on the bag body 10 attach to the periphery of the tray so that the tray is located in the enclosed accommodating cavity 10a and thus the endoscopes or other medical equipment located in the tray are located in the enclosed accommodating cavity 10a. With this arrangement, the plurality of connecting edges of the transport bag 100 all cover the tray so that a gap between the transport bag 100 and the tray opening can be avoided, thereby reducing the risk of contamination of the endoscopes or other medical equipment to be transported. As shall be appreciated, the first part 101 and the second part 102 are folded and connected with each other along the first folding side 10c, and the first folding side 10c constitutes one of the connecting edges of the transport bag 100.

Optionally, the bag body 10 is made of flexible materials, which may include one or a combination of polyethylene, polypropylene, polyvinyl chloride, polyester, polystyrene and polyamide. It shall be noted that the dimensions of the first part 101 and the second part 102 may not be specifically limited in the present application, and they can be set by users according to actual needs. For example, the widths of both the first part 101 and the second part 102 are 53 cm, the lengths of both the first part 101 and the second part 102 are 64 cm, the width of the bag opening 10b is 40 cm, and the length of the bag opening 10b is 50 cm. For another example, the widths of both the first part 101 and the second part 102 are 60 cm, and the lengths of both the first part 101 and the second part 102 are 70 cm.

In some embodiments, the plurality of first connection sides 1011 include a first upper side 1011a, a first lateral side 1011b and a first lower side 1011c, and the first lateral side 1011b is located between the first upper side 1011a and the first lower side 1011c; the plurality of second connection sides 1022 include a second upper side 1022a, a second lateral side 1022b and a second lower side 1022c, and the second lateral side 1022b is located between the second upper side 1022a and the second lower side 1022c. Moreover, the second upper side 1022a is connected with the first upper side 1011a, the second lateral side 1022b is connected with the first lateral side 1011b, and the second lower side 1022c is connected with the first lower side 1011c. With this arrangement, it can be ensured that the bag body 10 is formed with three connecting edges.

In some embodiments, the transport bag at least meets one of the following conditions: the first upper side 1011a and the second upper side 1022a are in hot-press sealing connection; the first lateral side 1011b and the second lateral side 1022b are in hot-press sealing connection; and the first lower side 1011c and the second lower side 1022c are in hot-press sealing connection. As shall be appreciated, the connection mode between the first upper side 1011a and the second upper side 1022a, the connection mode between the first lateral side 1011b and the second lateral side 1022b, as well as the connection mode between the first lower side 1011c and the second lower side 1022c are not limited to the above-mentioned hot-press sealing connection, but may also be realized by other ways, e.g., by gluing or the like.

In some embodiments, the first part 101 and the second part 102 are symmetrically arranged with respect to the first folding side 10c, so that after the first part 101 and the second part 102 are folded with each other, the first part 101 and the second part 102 can be attached to each other with basically the same attachment area, thereby forming a complete transport bag.

It shall be noted that, the medical equipment is divided into clean equipment and contaminated equipment, and thus during the transportation of the medical equipment with the transport bag 100, it is necessary to strictly distinguish the different equipment from each other so as to avoid confusion and mutual contamination there between. In the present application, in order to distinguish the clean equipment from the contaminated equipment, the bag body 10 is usually provided in different colors, which may be one or a combination of red, blue, green, yellow, orange, purple, black and white, as long as different equipment can be distinguished by the different colors. For example, the green bag body 10 represents the clean equipment placed in the tray, while the red bag body 10 represents the contaminated equipment placed in the tray.

In some embodiments, the bag body 10 is transparent, and the transport bag 100 is basically transparent. When the endoscope to be transported is not placed on the tray, a part of the transparent bag body 10 may be located in the tray after the transparent bag body 10 covers the tray, and then the endoscope to be transported and the transport bags 100 of other colors to be covered subsequently are placed. At this time, the transparent transport bag 100 can reduce the influence on the transport bags 100 of other colors, and at the same time, the transparent transport bag 100 can reduce the direct contact between the endoscope to be transported and the tray, thereby reducing the risk of contamination of the endoscope to be transported.

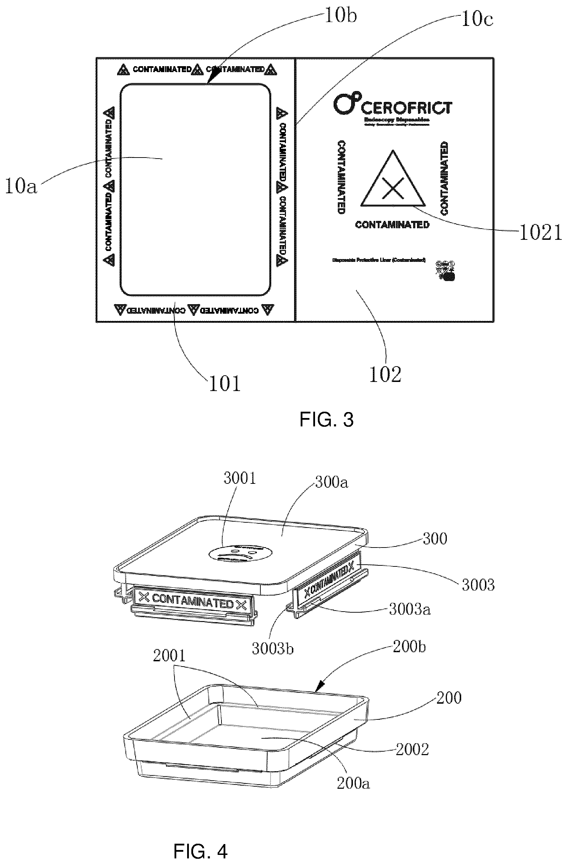

In some embodiments, referring to Figure 3 together, in order to distinguish the types of the equipment in the transport bag 100 more clearly, the outer surface of the second part 102 is further provided with a first identification symbol 1021, which can be used to distinguish the types of equipment in the transport bag 100. The first identification symbol 1021 may be different words, graphics, expressions or others. For example, when the first identification symbol 1021 is a smiling expression, it represents the clean equipment placed on the tray in the transport bag 100. When the first identification symbol 1021 is a crying expression, it represents the contaminated equipment placed on the tray in the transport bag 100. For another example, when the first identification symbol 1021 is a √ figure, it represents the clean equipment placed on the tray in the transport bag 100, and when the first identification symbol 1021 is a × figure, it represents the contaminated equipment placed on the tray in the transport bag 100. As shall be appreciated, the color of the transport bag 100 and the first symbol can be used together or independently, and they can be set by the users according to the actual needs, without specific limitation in the present application.

It shall be noted that, when the bag body 10 is transparent, different identification symbols may be correspondingly arranged on the inner side wall and the outer side wall of the bag body 10, and users can use the transparent transport bag 100 at the front side or the back side. For example, the inner side wall is provided with a smiling expression, while the outer side wall is provided with a crying expression. When clean equipment is placed on the tray, users can turn the transport bag 100 over so that the smiling expression on the inner side wall is exposed on the outer surface, and then cover the tray with the transport bag 100. When contaminated equipment is placed on the tray, the user can directly cover the tray with the transport bag 100, and at this time, the crying expression on the outer side wall is exposed on the outer surface.

In the embodiment of the present application, a bag body 10 is provided, and the bag body 10 is provided with an accommodating cavity 10a and a bag opening communicated with the accommodating cavity 10a. The bag body 10 includes a first part 101 and a second part 102 which are connected with each other, a first folding side 10c is provided between the first part 101 and the second part 102, and the first part 101 and the second part 102 are folded and connected with each other along the first folding side 10c. The bag opening is located on the first part 101, the first part 101 includes a plurality of first connecting sides 1011, the second part 102 includes a plurality of second connecting sides 1022, and one of the second connecting sides 1022 is correspondingly connected with one of the first connecting sides 1011 so that the bag body 10 forms a plurality of connecting edges. When users need to transport endoscopes or other medical equipment, they only need to put the endoscopes or other medical equipment to be transported on the tray, then cover the tray from the bag opening until the tray is located in the accommodating cavity 10a, and make the plurality of connecting edges on the bag body 10 attach to the periphery of the tray so that the tray is located in the enclosed accommodating cavity 10a and thus the endoscopes or other medical equipment in the tray are located in the enclosed accommodating cavity 10a. With this arrangement, the plurality of connecting edges of the transport bag all cover the tray so that a gap between the transport bag and the tray opening can be avoided, thereby reducing the risk of contamination of the endoscopes or other medical equipment to be transported.

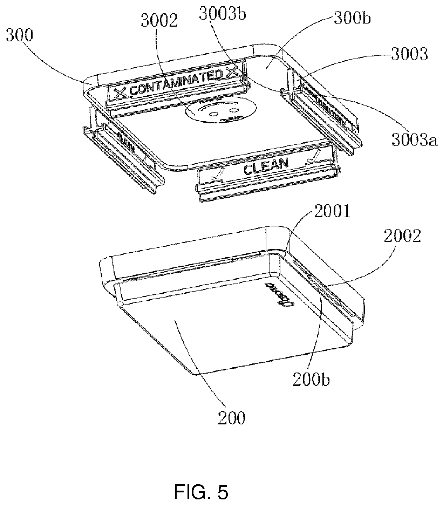

The present application further provides an embodiment of a transport box. As shown in Figures 4 and 5, the transport box includes a box body 200 and a cover plate 300. The box body 200 is provided with an accommodating space 200a and an opening 200b communicated with the accommodating space 200a. The cover plate 300 covers the opening 200b, and the periphery of the cover plate 300 is connected to the periphery of the box body 200. The endoscope or other medical equipment to be transported is placed in the accommodating space 200a from the opening 200b, and then the box body 200 is covered with the cover plate 300 to seal the accommodating space 200a, so as to reduce the entrance of external dust or other impurities into the box body 200, thereby reducing the risk of contamination of the endoscope or other medical equipment to be transported. As shall be appreciated, the endoscope or other medical equipment to be transported may also be placed on the tray, and then the tray is placed in the accommodating space 200a from the opening 200b to be covered by the box body 200, and then the box body 200 is covered and sealed by the cover plate 300. In this way, the tray can reduce the direct contact between the endoscope or other medical equipment to be transported and the transport box, thereby reducing the risk of contamination of the endoscope or other medical equipment to be transported.

In some embodiments, the cover plate 300 includes a first surface 300a and a second surface 300b which are oppositely arranged. When the cover plate 300 covers the opening 200b, the first surface 300a or the second surface 300b is located in the accommodating space 200a. The cover plate 300 can cover the opening 200b with any of the two different sides so that the first surface 300a or the second surface 300b on the cover plate 300 is exposed. Optionally, the first surface 300a is provided with a second identification symbol 3001, and the second surface 300b is provided with a third identification symbol 3002. When the first surface 300a is exposed, the second identification symbol 3001 is exposed, and when the second surface 300b is exposed, the third identification symbol 3002 is exposed. As shall be appreciated, different identification symbols exposed represent different types of endoscopes located in the box body 200, and the two different identification symbols correspondingly represent different types of endoscopes. For example, the second identification symbol 3001 is a smiling expression to indicate that the endoscope located in the box body 200 is in a clean state, while the third identification symbol 3002 is a crying expression to indicate that the endoscope located in the box body 200 is in a contaminated state. The second identification symbol 3001 and the third identification symbol 3002 may be different words, graphics, expressions or others.

It shall be noted that, the user can turn over the cover plate 300 according to actual needs so that the second identification symbol 3001 or the third identification symbol 3002 is exposed. That is, one side of the cover plate 300 is provided with a mark indicating cleanliness, while the other side of the cover plate 300 is provided with a mark indicating contamination. For example, when the second identification symbol 3001 indicates that the endoscope located in the box body 200 is in a clean state and the third identification symbol 3002 indicates that the endoscope located in the box body 200 is in a contaminated state, the transport box with the second identification symbol 3001 exposed is transported to a preset position, and after the endoscope is used by medical personnel, the endoscope is placed in the box body 200 again. At this time, the medical personnel need to turn over the cover plate 300 so that the third identification symbol 3002 is exposed, which is convenient for identifying different types of endoscopes. Moreover, the medical personnel only need to turn over the cover plate 300 instead of replacing the transport box, which is time-saving, labor-saving and convenient.

As shall be appreciated, the second identification symbol 3001 and the third identification symbol 3002 may also be used together with different colors or used independently. For example, the second identification symbol 3001 is a green smiling expression to indicate that the endoscope located in the box body 200 is in a clean state, while the third identification symbol 3002 is a red crying expression to indicate that the endoscope located in the box body 200 is in a contaminated state.

In some embodiments, the periphery of the box body 200 extends with a stepped part 2001 in the direction away from the accommodating space 200a, and one side of the stepped part 2001 away from the cover plate 300 is provided with a clamping protrusion 2002, a clamping groove 200b is arranged between the clamping protrusion 2002 and the outer wall of the box body 200, and the clamping groove 200b is used to cooperate with the members of the cover plate 300 so as to make the cover plate 300 closely locked onto the box body 200 to ensure the tightness of the accommodating space 200a.

In some embodiments, the periphery of the cover plate 300 is further provided with a fastening plate 3003, the fastening plate 3003 is rotatably connected to the cover plate 300 and can rotate relative to the cover plate 300, one side of the fastening plate 3003 close to the first surface 300a is provided with a first hook 3003a, and the other side of the fastening plate 3003 close to the second surface 300b is provided with a second hook 3003b. As can be known from the above description, the cover plate 300 can cover the opening 200b with any of the two different sides so that the first surface 300a or the second surface 300b on the cover plate 300 is exposed. When the first surface 300a is located in the accommodating space 200a, the second surface 300b is exposed, the first hook 3003a is clamped in the clamping groove 200b and the first hook 3003a abuts against the clamping protrusion 2002, thereby closely locking the cover plate 300 onto the box body 200. When the second surface 300b is located in the accommodating space 200a, the first surface 300a is exposed, the second hook 3003b is clamped in the clamping groove 200b, and the second hook 3003b abuts against the clamping protrusion 2002, thereby closely locking the cover plate 300 onto the box body 200.

What described above are only the embodiments of the present disclosure, but are not intended to limit the scope of the present disclosure. Any equivalent structures or equivalent process flow modifications that are made according to the specification and the attached drawings of the present disclosure, or any direct or indirect applications of the present disclosure in other related technical fields shall all be covered within the scope of the present disclosure.

## Claims

1. A transport bag, being **characterized in that**, comprising:
a bag body, being provided with an accommodating cavity and a bag opening communicated with the accommodating cavity;
wherein the bag body comprises a first part and a second part which are connected with each other, a first folding side is provided between the first part and the second part, the first part and the second part are folded and connected with each other along the first folding side, the bag opening is located on the first part, the first part comprises a plurality of first connecting sides, the second part comprises a plurality of second connecting sides, and one of the second connecting sides is correspondingly connected with one of the first connecting sides so that the bag body forms a plurality of connecting edges.

2. The transport bag according to Claim 1, being **characterized in that**, the plurality of first connecting sides include a first upper side, a first lateral side and a first lower side, the first lateral side is located between the first upper side and the first lower side;
wherein the plurality of second connecting sides include a second upper side, a second lateral side and a second lower side, the second lateral side is located between the second upper side and the second lower side, and the second upper side is connected with the first upper side, the second lateral side is connected with the first lateral side, and the second lower side is connected with the first lower side.

3. The transport bag according to Claim 2, being **characterized in that**, the transport bag at least meets one of the following conditions:
the first upper side and the second upper side are in hot-press sealing connection;
the first lateral side and the second lateral side are in hot-press sealing connection; and
the first lower side and the second lower side are in hot-press sealing connection.

4. The transport bag according to Claim 1, being **characterized in that**, the first part and the second part are symmetrically arranged with respect to the first folding side.

5. The transport bag according to Claim 1, being **characterized in that**, the outer surface of the second part is further provided with a first identification symbol.

6. The transport bag according to Claim 1, being **characterized in that**, the bag body is made of flexible materials.

7. A transport box, being **characterized in that**, comprising:
a box body and a cover plate, wherein the box body is provided with an accommodating space and an opening communicated with the accommodating space, the cover plate covers the opening, and the periphery of the cover plate is connected with the periphery of the box body.

8. The transport box according to Claim 7, being **characterized in that**, the cover plate comprises a first surface and a second surface which are oppositely arranged, and when the cover plate covers the opening, the first surface or the second surface is located in the accommodating space.

9. The transport box according to Claim 8, being **characterized in that**, the first surface is provided with a second identification symbol, and the second surface is provided with a third identification symbol.

10. The transport box according to Claim 9, being **characterized in that**, the periphery of the box body extends with a stepped part in the direction away from the accommodating space, and one side of the stepped part away from the cover plate is provided with a clamping protrusion, and a clamping groove is arranged between the clamping protrusion and the outer wall of the box body;
wherein the periphery of the cover plate is further provided with a fastening plate, the fastening plate is rotatably connected to the cover plate and can rotate relative to the cover plate, one side of the fastening plate close to the first surface is provided with a first hook, and the other side of the fastening plate close to the second surface is provided with a second hook, wherein when the first surface is located in the accommodating space, the first hook is clamped in the clamping groove, and the first hook abuts against the clamping protrusion; and when the second surface is located in the accommodating space, the second hook is clamped in the clamping groove, and the second hook abuts against the clamping protrusion.
